# EUROPEAN PATENT SPECIFICATION

(11) **EP 1 545 429 B1**
(45) Date of publication and mention of the grant of the patent: **29.12.2010**
(21) Application number: 03772113.1
(22) Date of filing: 29.07.2003
(51) Int. Cl.: A61K 31/194, A61K 9/70, A61K 9/00, A61K 47/00, A61P 17/00, A61P 31/10

(54) **COMPOSITIONS CONTAINING CITRIC ACID FOR THE TREATMENT OF NAIL FUNGAL INFECTIONS**
ZUSAMMENSETZUNGEN ENTHALTEND ZITRONENSÄURE ZUR BEHANDLUNG VON NAGELPILZERKRANKUNGEN
COMPOSITIONS CONTENANT DE L'ACIDE CITRIQUE POUR LE TRAITEMENT DES INFECTIONS FONGIQUES DE L'ONGLE

(30) Priority: 29.07.2002 US 207936
(43) Date of publication of application: 29.06.2005
(73) Proprietor: ACRYMED, INC., Portland, OR 97223 (US)
(72) Inventor: MALEY, Joseph, Sunriver, OR 97707 (US); GIBBINS, Bruce, Lake Oswego, OR 97035 (US)
(74) Representative: Harrison, Ivor Stanley
(86) International application number: PCT/US2003/023851
(87) International publication number: WO 2004/010952

(56) References cited:
- WO-A1-96/11572
- WO-A2-00/15202
- US-A- 5 181 914
- US-A- 5 196 190
- US-A- 5 464 610
- US-A- 5 853 742
- US-A- 5 961 996
- US-A- 5 993 790
- US-A- 6 110 447
- US-A- 6 143 794
- US-B1- 6 231 840
- US-B1- 6 270 811

## Description

### Field of the Invention

This invention discloses compositions for use in the treatment of nail fungal infections. Particularly, this invention relates to treatment of nail fungal infections using topical delivery vehicles as specified in the claims to provide active agents to the site or sites of pathology.

### Background of the Invention

The dermal structures of humans and other animals are often the site of infections or attachments by other living organisms. Dermal structures include skin, hair, hair follicles, cornea, sclera, organ linings, pleural coverings, dura, toenails, fingernails, hooves, horns, mucous membranes, and other cellular structures made from epithelial cells or keratinized structures. Organisms that live in or on the dermis or dermal structures of humans and animals include microorganisms such as yeasts, fungi, bacteria, viruses, mycoplasma, and insects such as dust mites, ticks, lice and other arthropods.

The presence of these organisms in and on dermal structures often cause a range of changes, from merely unsightly to pathological conditions, to the dermis and dermal structures, and can interfere with the functions of the dermis and dermal structures of the host organism. Additionally, the presence of these organisms can result in immunological responses by the host and cause secondary problems at the site or a general response throughout the entire host organism. Thus, there is a need to prevent infection or attachment by such organisms, control the amount of infection or attachment by such organisms, treat the affected sites, and prevent the re-infection and re-attachment by such organisms from the environment.

One of the most common and difficult to treat dermal conditions is infection of nail structures. The finger and toenails of humans are specialized tissue structures that are closely related to claw and hoof tissues in animals. The nails constitute an important protective structure at the distal ends of the fingers and toes that shield the highly sensitive extremities from triggering pain or sensation on contact with the external environment. Nails also function as an important tool that is used to increase the range of dexterous accomplishments. Humans have developed great pride in the function and appearance of nails. In many cultures, painting and decoration of the nails of both the hands and feet are common place.

Nails, like most anatomical structures of the body, are susceptible to disease processes. One of the pathological conditions that is common for humans is caused by microbial invasion of the nail or surrounding tissues. Although the condition is not life threatening, infection of these tissues can compromise the appearance and the function of the affected nail. One such condition results from the invasion of the tissues by one or more of several fungi. Fungal involvement in nail infection has been termed onychomycosis.

Onychomycosis, or fungal infection of the nail, is the most common cause of nail dystrophy. The incidence of the condition has been difficult to determine largely because it is not life threatening and rarely is reported. It has been estimated that the incidence ranges from 3% to 23% of the total population in western cultures. There is no clear understanding of the circumstances that predispose to this condition. It has been suggested that there is a relationship between trauma to the nail region and the occurrence of infection. However no studies have confirmed this hypothesis.

It is known that certain lifestyle and climatic situations seem to contribute to onychomycosis. One example is the greater than 80% occurrence rate of onychomycosis in the general population in outlying northeastern areas of Russia where long cold winters are encountered. This is likely due to the necessity of using heavy protective footwear for prolonged periods to protect the feet from the cold. This exposes the foot, and more particularly the nails, to prolonged moist warm conditions that may encourage the growth of the fungi.

In western cultures it is well known that there is an age related distribution in the incidence of onychomycosis. The condition is much more prevalent in elderly individuals. Indeed it has been estimated that 80 to 90% of the elderly in the U.S. and Canada have at least one or more of their nails involved. One reason for the increase with age is that onychomycosis poorly responds to treatment, hence is seldom treated so there is an accumulation of cases. Other factors that may contribute to the increase with age are a general waning of innate and specific immunity to fungal pathogens as well as an increased accumulation of co-morbidities that may contribute to invasion by the fungal pathogens associated with onychomycosis.

Onychomycosis is a dystrophic condition of the nails of the feet and hands. Fungal infection of the nails results in characteristic changes in the appearance to the structure. The appearance is often used to describe the degree of severity of the infection. Clinicians often use appearance alone in differential diagnosis of the condition. Knowledge of the state of the disease is often useful in determining the course of treatment and the likelihood of success in the management of the disorder. The common forms of the disease are listed in Table 1.

**Table 1**

| State of disease | Tissue involve | Severity |
|---|---|---|
| Distal Lateral Subungual Onychomycosis (DLSO) | Fungal invasion at the extreme distal end of the nail bed. | Superficial |
| Proximal Subungual Onychomycosis (PSO) | Fungal invasion at the proximal end of the nail bed. | Superficial to severe. Most often in immunocompromised people. |
| White Superficial Onychomycosis (WSO) | Nail plate only resulting in discrete to extensive white patches or streaks in the nail. | Superficial to severe. |
| Total Dystrophic Onychomycosis (TDO) | Entire nail bed involved. | Severe. |
| Endonyx | Partial or complete involvement of nail plate. | Superficial to severe |

These different presentations are important in predicting the outcome of a treatment strategy. The anatomy of the nail and its supporting structures profoundly influence the effectiveness of treating the tissues to rid them of the fungal pathogens. The reason for this is that nail structure and anatomy are complex and, to some degree, are isolated from systemic circulation. Furthermore the nail plate is relatively impermeable to penetration by topical agents.

The anatomy of the human nail is important for the understanding of the process that results in onychomycosis. The nail unit is a form of specialized epidermal tissue. The nail apparatus, which comprises all the elements colloquially referred to as "the nail", is composed of distinct structural elements. The most prominent structure of the nail apparatus is referred to as the nail plate which is a thin hard flexible structure that emerges from skin folds at the extremities of the digits. The nail plate is produced by the nail matrix situated in the proximal fold. The matrix causes nail plate elongation through proliferation of its epidermal-like cells. The lateral folds hold the nail plate in an orientation so that elongation proceeds towards the distal end of the extremity. The nail plate grows at the rate of 1 mm (toe nail) to 3 mm (fingernail) per month. The nail plate is translucent in appearance, almost always convex in shape and approximately 0.2-0.6 mm thick. It is composed of approximately 25 layers of dead, keratinized cells that originate from the matrix. These dead cells are held tightly together by intercellular linkages resulting in a coalescent dense hard plate with a smooth dorsal surface. The ventral side of the nail plate is relatively irregular which facilitates attachment of collagen fibers which serve to anchor the nail plate to the nail bed. Nail plate elongation continues toward the distal end of the digit where it parts from the nail bed at a point termed the hyponychium.

The nail plate, chemically, is largely protein along with approximately 10-30% aqueous moisture. Moisture content in the nail plate is directly proportional to the moisture content of the environment. Immersion in water will increase moisture content as will occlusion of the surface so as to prevent evaporation. The nail plate also contains between 0.1 to 1% lipid. There is no central circulation or lymphatic drainage associated with the nail apparatus. However the tissues proximal and lateral, and those beneath the nail bed are highly vascularized. Notwithstanding, the matrix is the only viable component of the nail and is the only structure supported by systemic circulation. The remainder of the nail apparatus is non-viable and is separated from systemic support by a layer of collagen fibrils and nail plate substrate.

Under normal circumstances the nail plate and hyponychium present a formidable barrier to microbial penetration. Indeed, most cases of onychomycosis originate in the distal area of the nail which implies a disturbance to the tight junction at the hyponychium. This probably provides fungi access to the region beneath the nail plate that is rich in organic substrate sufficient to support the growth of the fungi. Less frequent, is the direct fungal penetration into the nail plate. *Trychopyton mentagrophytes* can invade in this way because it produces enzymes that are capable of degrading the protein matrix of the nail plate, thereby enabling the fungus to penetrate directly into the substrate. In either case the fungi enter a privileged region that is largely devoid of natural resistance mediated by the immune system since there is no systemic circulation to the tissues. Moreover, the absence of circulation also restricts the ability of delivering systemic anti-microbial agents effectively to the specific sites where the fungi inhabit.

Onychomycosis is an active disease process that involves the growth and replication of saprophytic fungi in the nail apparatus. Most often the fungi are in the non-viable region between the nail plate and the nail bed. Accurate diagnosis of the condition requires the microscopic examination and culturing of specimen material taken from the affected region. Typically scrapings from the site of infection are first digested in a strong solution of KOH, mounted on microscope slides with a suitable stain specific for fungal mycelium, and then visualized for typical structures common to dermatophytes. In addition, differential diagnosis is greatly enhanced by propagation and identification of fungi using in vitro culturing methods. However, microscopic examination and in vitro culture are time consuming and expensive. Therefore many clinicians will make an imperical diagnosis based on the appearance of the affected nails, lifestyle factors, and age of the individual. This approach may lead to a misdiagnosis of cases resulting in inappropriate and dangerous treatment modalities. There is need for a treatment that is safe and economical that can be implemented with little or no risk should the original diagnosis be incorrect.

The current systemic and the topical methods for the management of onychomycosis are expensive, have low success rates and in many cases expose the user to considerable risk of central organ damage. This is predominately due to the need to deliver sufficient active agent to the individual to achieve an inhibitory concentration in the non-viable regions where the fungi resides. In most cases delivery and accumulation of the active agent is dependent upon diffusion through non-viable and non-vascularized substrate (nail plate, or anchor protein zone of the nail bed). This often has meant persistent administration of the agent with its concomitant risk of systemic toxicity.

The alternative to a direct attack on the fungi is the removal of its privileged habitat. This can be accomplished by removal of the nail plate which provides both nutrient substrate and protection to the fungal parasite. Surgical ablation is still recommended by some as quicker, and better accepted than chemical ablation which is essentially painless. Others condemn it and say chemical ablation is the only way it should be done. Chemical ablation is done by covering the skin around the nail for protection and then applying 40% urea to the nail. An occlusive dressing is then put on and left for a week at which time the patient returns to the doctor for removal. The expense includes a minimum of two doctor visits plus the "procedure" cost. It is effective at removing the nail but it is known to have a fairly high failure rate unless combined with drug treatment. Furthermore it requires between 6 months to a year for the complete outgrowth of new nail plate and a return to a normal appearance of the nail apparatus. It is also somewhat controversial as to its effectiveness as there are no studies showing actual incidence of cure.

The use of oral drugs has lead to the achievement of excellent penetration and accumulation of active agents within the nail after prolonged (months) duration of drug administration. Topical administration has also resulted in good penetration and accumulation of the active. Drug levels have been recorded by direct assay for the active agents, yet in both topical and oral administration there has been a dismal record of cure rate. Complicating evaluation of true cure rate is that "cure" has been ill-defined. In some studies it is taken to mean the achievement of a normal looking nail apparatus whereas in other cases it is taken to mean the elimination of microscopic or culture detectable fungi in specimens collected from the treatment site. Regardless of the method of assessment there is clear recognition that the high cost of treatment, the poor level of outcome, and the concomitant risk of side effects from the therapy have identified that there is a need for a safe, effective, and economic treatment modality for onychomycosis.

There is also a need for treatments for other dermal conditions that are easily used and applied by patients because the treatment times are often long and extended. An ideal treatment modality for onychomycosis and other dermal pathologies would be effective and sufficiently straightforward so that it encourages a high degree of patient compliance. A simple and relatively short duration of administration of an agent targeted for the organism responsible for the pathology should be highly effective at controlling or eliminating the disease-state. Effective topical treatment of the conditions requires successful penetration of the active agent through the nail plate or dermal layers to the zone most commonly harboring the organism. An effective treatment would include the penetration and accumulation/maintenance of an effective concentration of active agent in the growth site and habitat of the unwanted organism.

Compositions are needed that are effective for treatment of unwanted organisms causing pathological conditions in the skin and dermal structures. Such compositions can be used in methods for preventing infection or attachment, treating existing conditions, and preventing re-infection or re-attachment of organisms in the environment. Such compositions should be well tolerated by the patients.

US6143794A teaches a topical antifungal composition including a gel carrier for a specific antifungal compound. In addition to the antifungal compound and its solvent, the composition includes a gel that consists of organic macromolecules distributed throughout the composition. The composition may be squeezed from a tube similar to a toothpaste tube or painted on with a brush.

US5993790A teaches a water-based nail lacquer and urea composition. The composition is a liquid that may be applied to nails.

US5091171A teaches amphoteric or pseudoamphoteric compounds with hydroxy acid compositions that may be formulated into a solution, gel, cream, ointment or shampoo and teaches that, for nail treatments, the composition is applied as a small drop using a toothpick or artist's brush.

### Description of the Invention

According to the present invention, a composition for use in treating nail fungal infections comprises
a) a matrix sheet comprising a natural or synthetic hydrophilic polymer;
b) one or more active agents, wherein at least one active agent is citric acid;
c) at least one humectant; and
d) a water content of 0.1-50%.

As used herein, skin, dermal or dermal structures refers to the elements of the integumentary system of humans and animals, including organ and cavity linings, such as pleura, dura, and pericardium, and mucous membranes and such terms are herein used interchangeably. The integumentary system as it is commonly understood by those skilled in the art refers to the skin, including the epidermis and dermis, which comprise layers including stratum basale, stratum spinosum, stratum granulosum, stratum lucidum, stratum corneum, papillary layer and reticular layer. Cells which are found in these dermal structures include, but are not limited to, keratinocytes, fibroblasts, melanocytes, dendritic cells, Landerhans' cells, epithelial cells, and Merkel cells. Dermal structures include appendages such as hair, sebaceous glands, sweat glands, follicles, nerve structures, horns, hooves and nails.

One embodiment of the invention comprises compositions for use in treating fungal mediated nail infections referred to as onychomycosis. A composition comprises a matrix material that comprises one or more therapeutic agents, latent moisture, one or more humectants, and may further comprise means to secure the matrix in direct contact with a nail plate.

Though not wishing to be bound by any particular theory, it is thought that a delivery vehicle such as a polymerized matrix structure would provide a reservoir comprising one or more active agents, water and a humectant composition that will be delivered to the nail plate when secured to the nail. The moisture and humectant that are transferred on application to the nail will hydrate the nail plate and form a diffusion gradient for the effective delivery of the active agent to the non-viable areas of the nail apparatus.

Dermal structures are infected by or provide a growth site for a variety of organisms. Disclosed is treatment of dermal structures affected by such organisms. Such organisms include organisms that live in or on the epidermis, dermis or dermal structures of humans and animals including microorganisms such as yeasts, fungi, bacteria, viruses, mycoplasma, and insects such as dust mites, ticks, lice and other arthropods.

Organisms termed fungi include a diverse group of free living organisms that include molds, smuts, mushrooms, mildews and yeast. Collectively these organisms are referred to as heterophils, meaning that they utilize non-living complex organic substances as their source of nutrients. These organisms play an important role in the recycling of dead and decaying organic matter in nature. A few fungi have adapted to a saprophytic relationship with higher organisms such as man. Even fewer of these are capable of invading to establish in the unique ecological zone of the nails of mammals. Although a diverse number of fungi have been described in association with nail dystrophy and other dermal pathologies, the vast majority of cases in the U.S. and Canada are caused by a group of fungi known as dermatophytes. Dermatophytes have one property in common; that being that they all are capable of invading and establishing residence in the tissues associated with the skin. Often fungal infection of the skin is referred to as ringworm and the phrase "ringworm of the nails" has been used to describe onychomycosisis. The principle nutrient source for dermatophytes in skin and nails is keratinized proteins. Keratin is non-viable matrix protein complexes derived from epidermal tissues. Typically dermatophytes do not invade beyond the non-viable keratinized layers. Although dermatophytes constitute 90 - 96% of the cases of onychomycosis, other fungi such as yeasts and molds have also been found in association with the disease.

The predominate isolates from infected nails has been *Trychophyton rubrum* followed thereafter by *Trychophyton mentagrophytes, Epidermophyton sp.* and *Microsporum sp.* Non-dermatophyte molds that are occasionally isolated include *Aspergillus niger* and *Scopulariopsis brevicaulis.* Collectively these less frequently encountered fungi account for less than 4 -5% of cases. Even less frequently encountered are yeast. The most common yeast isolate is *Candida albicans* which accounts for 3 - 4% of the total cases. Interestingly, the *C. albicans* onychomycosis is more frequent in women than in men, most often affecting their fingernails.

The cell wall of fungi is primarily chitin, a natural long chain polysaccharide composed largely of poly-(N-acetyl glucosamine). Chitin is extensively distributed in nature, making up the predominate component of the exoskeletons of crabs, spiders and many other creatures. The cell membranes of fungi are similar to the cell membrane structure of human cells except that fungal cell membranes contain ergosterol and zymosterol unlike the cholesterol found in mammalian cell membranes. This is an important difference that has allowed the targeting of specific anti-fungal treatments. All current anti-fungal agents are based on disrupting the biosynthesis of these sterols.

The biosynthesis of squalene to ergosterol is depicted as:
Squalene → Squalene epoxide → Lanosterol→14 Dimethyl Lanosterol→ Zymosterol→Fecosterol→Episterol→Ergosterol.

There are several anti-fungal compounds that have been extensively evaluated for their activities against fungi and in particular, the dermatophytes which are associated with onychomycosis. TERBENAFINE is an allylamine which blocks the conversion of squalene to squalene epoxide. This results in the accumulation of squalene in the cell. Squalene is an important intermediary compound in ergosterol synthesis but in higher concentrations becomes toxic for the fungi as it accumulates inside the cytoplasm. Lamisil® is the trade name for terbinafine and is considered the most effective oral medication for onychomycosis. It has an advantage over others in that it is also the least likely to cause serious side effects following prolonged usage. Its basis for effectiveness is that it is fungicidal whereas the other agents are fungistatic. TERBINAFTNE is less effective for yeast and to some extent molds. It persists for several months after treatment and can be detected in the nail bed at one week. Clinical cure rate is 37% of cases of the total dystrophic onychomycosis (TDO) form of infection. Serious side effects include liver damage and severe generalized skin reactions such as the Stevens-Johnson's syndrome requiring hospitalization. While serious side effects are not common, there were 11 deaths and 2 liver transplants reported to the US government by 2001 out of 16 cases of liver failure. At a 37% cure rate for TDO of the great toe and typically six months treatment required, the overall cost per cure is around $6,500.

Azol antifungals such as ketoconazole, itraconazole, and fluconazole all block the conversion of the intermediate, lanosterol to 14-dimethyl lanosterol. This process starves the cell of intermediates for the synthesis of ergosterol. SPORANOX is the trade name for itraconazol which has been used for the treatment of onychomycosis. It has been generally shown to be less effective than TERBINAFINE in most studies, both in rate of cure and in the relapse rate. It is commonly given as a "pulse", i.e., one week on and three weeks off the oral medication. It is more effective than TERBINAFINE against molds and probably against yeast forms but less effective against dermatophytes. Clinical cure rate with TDO is about 27%. There is a probable association of pulmonary edema with SPORANOX with 58 cases as of 2001 including 13 deaths reported. There were 24 cases of liver failure with 11 deaths from SPORANOX reported to the FDA through 2001. If given daily, the cost is the same as LAMISIL at about $10/tablet or capsule. The cost is less if "pulse treatment" is used. SPORANOX requires regular liver enzyme function monitoring to provide early detection of hepatic injury due to the therapy as is the case for LAMISIL. DIFLUCAN (fluconazole) commonly used for vaginitis is particularly effective against yeast forms (Candida). It persists in the nail plate for several months after treatment. The cure rate for total dystrophic onychomycosis (TDO) is about 38%. It, like LAMISIL and SPORANOX, is also approximately $10/pill and is a once per day dose but can be used with "pulse treatment" like itraconazole. Ketoconazole and Griseofulvin are no longer recommended for onychomycosis.

Amorolfine blocks conversion of intermediates at two sites in the synthetic pathway. It blocks both at the conversion of 14-dimethyl lanosterol to zymosterol and at the conversion of fecosterol to episterol. Again this has the effect of limiting cell membrane synthesis and leads to the selective killing of the fungi dependent upon this pathway. LOCERYL is an amorolfine that is used topically as a 5% solution and is approved for use in Europe for treatment of onychomycosis. It is indicated for mild onychomycosis with no matrix involvement and is effective in about 40% of the cases.

PENLAC (ciclopirox olamine) is the only FDA approved topical treatment for onychomycosis. It is a topical anti-fungal that is painted on the nail, typically once or twice a day for a year or more. It has been recommended for use in combination therapy involving topical plus oral application. In vitro, it is fungicidal against dermatophytes, yeast and molds that typically cause onychomycosis. In a study of mild to moderate distal lateral subungual onychomycosis (DLSO) form of the disease (an average of 40% of nail involved and no matrix infection), it had a 7% cure rate. At $420 per treatment for the drug cost, coupled with a 7% cure rate, extrapolates a cost per cure of almost $8,500 presuming three office visits per patient.

Many individuals with nail dystrophic conditions have turned to alternative concoctions in search of a cure. Tea tree oil (Melaleuca alternafolia) has been claimed to be effective as a "natural" treatment. The only clinical study available is one using it as the "control". There were no cures in the control group of that study. Other alternative treatments advertised include Varisi (50% citrus seed extract, 25% ascorbic acid and 25% glycerin) and Nonyx Gel (acetic acid 9.75%) topically applied daily. Again there is little controlled clinical study information available for either of these and they logically would be unlikely to be effective.

Anti-fungal agents are specifically targeted to interfere with metabolic function of fungi and this can be repeatedly demonstrated under laboratory conditions. However known agents delivered either topically or systemically or, in some cases, as a combination have a low success rate. In theory, these drugs should have a cure rate that approaches 100% success. Some of the factors contributing to the low success rate are:
1. Nail matrix involvement in the condition where it is difficult to penetrate and deliver topical agents at effective concentrations.
2. Penetration of the anti-fungal into the areas were it can be active in controlling fungal growth is problematic. The lateral borders of the nail are exceptionally difficult regions to cause the accumulation of effective levels of actives regardless of whether oral agents or topical agents are used.
3. Presence of a dermatophytoma, a thick mass of keratin debris between the nail plate and bed, which contributes to poor penetration of either oral or topical drugs into the nail apparatus.
4. Progression of the dystrophic condition to a state known as oncholysis whereby the nail plate detaches from the nail bed resulting in poor penetration and accumulation of oral or topical drugs.
5. There are at least 21 variants of *T. rubrum* which is the predominant causative parasite of onychomycosis. It is possible that some may be more resistant to some treatments.
6. Fungal arthrospores are a dormant state of the fungi and are relatively resistant to anti-fungal agents.
7. An individual's compliance to a treatment regime is singularly difficult to maintain and is likely a major contributor to poor success rates with all current modalities. All current treatments require long periods with (usually) daily intervention to accomplish resolution of the infection. One survey showed a 48% incidence of a failure to adhere to the treatment schedule and 25% of individuals stopped completely before the end of scheduled therapy, often because they thought they were cured and didn't need it any more even though they did.
8. Ineffective elimination of fungi from vectors such as footwear, flooring and bathing surface, and keratinized skin layers results in continuous recurrent introduction of the fungi.

The nail apparatus presents unique challenges. Penetration of active substances, regardless of whether it is from systemic or topical application is dependent upon the movement of the agent through non-viable tissues where the fungi reside. It is likely that the most effective method of distribution through this type of environment would be through diffusion. Moisture or lipid provide the only fluid phase components in the nail apparatus that can support diffusive distribution of the agent. Unfortunately neither of these fluid phase components is in abundant concentrations within the nail plate and nail bed substrates to be exploited for effective delivery of actives. Furthermore the typical infected nail plate is usually considerably thickened in response to fungal activity and therefore presents a greater barrier to diffusion through the small amount of normal latent moisture in the nail.

Fortunately the nail plate is hydrophilic and will readily absorb moisture into the substrate. Therefore either exposure to moisture or, conversely, the use of agent(s) that attract moisture from the surrounding environment can substantially increase the moisture content of the nail plate. This would then provide a diffusion gradient for the transport of an antifungal agent into the sub-plate matrix to act on the fungi. It is known that penetration of compounds through the nail plate can be considerably enhanced by increasing the moisture content in the plate matrix. Therefore a device that incorporates both the active agent(s) and a moisture management system in a reservoir fashion would represent an improvement on previous devices that have been described for aiding in the treatment of onychomycosis.

The majority of onychomycosis involves invasion of the fungi into the bed of the nail. The exceptions are associated with some yeast and molds, and most of the cases secondary to *Trychopyton mentagrophytes* involve the actual nail plate. The invasion of the hard, devitalized keratin structure of the nail plate is assisted by enzymatic degradation of the substrate. This capability is most notable in *T. mentagrophytes* which has enzymes that allow it to digest the nail plate which opens a tract for the saprophyte to gain access into the deeper regions of the structure. Other organisms may also follow its course to establish a co-incident infection below the surface. In advanced stages ofonychomycosis both the plate and bed are usually involved. While permanent injury to the bed or matrix will result in permanent loss of the nail plate, most fungal infections do not cause permanent damage. With resolution of the infection, a normal nail can usually be restored by outgrowth of new nail plate structure.

There is a clear differential in the distribution of the disorder between toe and finger nails. Toenails are at least four times more commonly involved than fingernails. One reason may be that the warm, moist environment provided by shoes and stockings is optimal for the growth of dermatophytes. Other factors that may contribute to the increased incidence in toenails is that they are slower growing, are less frequently subjected to cleansing practices, and are continually assaulted with fungi that inhabit footwear. This is supported by the finding that onychomycosis is co-incident with dermatophyte infections elsewhere on the individual. Approximately a third of people with tinea pedis, or athletes' foot will develop onychomycosis.

Adjuvants to attempt enhanced delivery of active agents through the nail plate are not novel. U.S. Patents 5,840,283 and 5,972,317 describe the use of a proteolytic enzyme in conjunction with a medication for the treatment of nail fungus. Moisture may be another useful agent for facilitating the movement of active agents through the nail plate.

Compositions of the present invention include a humectant which will attract aqueous moisture from the atmosphere as well as that which is transpired normally into the nail plate. An embodiment of a humectant of the present invention would be of sufficient low molecular size to penetrate and localize in the nail plate so that moisture attracted to the humectant will also localize in the nail.

Numerous organic compounds and salts act as humectants or water retention agents. Humectants may include any of several organic alcohols such as glycerol, butanol, propanol, isopropyl alcohol, ethanol, methanol, propylene glycol, polyethylene glycol, ethylene alcohol, and butyl alcohol. In addition, several salts, including sodium chloride, lithium chloride, copper chloride, magnesium chloride, magnesium sulfate, mangenese sulphate, aluminum sulfate, zinc sulfate, and zinc chloride may function as water retention agents to assist in the hydration of the matrix. In the present invention, the preferred humectant is an organic alcohol since these are well tolerated by tissues yet are of sufficiently small molecular size as to readily migrate with the moisture into the nail plate matrix. The most preferred organic alcohol is glycerol, in concentrations ranging from 4 to 40 percent total weight of the composition. Though not wishing to be bound by any particular theory, it is believed that the humectant is helpful to the composition as it serves to both attract and cause the deposition of moisture in the composition as well as to preserve the aqueous moisture component to prevent loss by evaporation. It is intended that embodiments of the invention, when placed and secured to the nail plate, will deliver aqueous moisture into the plate to form the diffusion gradient of the active agent in the material. Equilibrium of distribution should then result in uniform concentration throughout the composition of the invention and the nail plate. It is also known that increased hydration of the nail plate increases the permeability of materials of varying molecular weights through that substrate. Therefore an added benefit of the incorporation of a humectant such as an organic alcohol, an example being glycerol, would assist in the penetration of agents of a variety of molecular sizes through the nail plate.

The present invention comprises compositions comprising a matrix that will provide molecular space for the incorporation of one or more active agents along with the moisture and one or more humectants during production of the product. Moreover, the composition of the material should be suitable for delivering the material from the matrix to the nail plate upon application during treatment. A suitable component would be any component that is composed of a flexible material that will conform to the contours of the nail. The material also should be capable of containing an effective amount of aqueous moisture to form a diffusion gradient between the matrix and the site of application, when applied, as well as comprise the other components of the delivery system until they function during the application.

Numerous substrates may be suitable for matrices. These include natural and synthetic hydrophilic polymers that may include rubber, collagen, animal hide, hyaluronic acid, dextran, alginates, cellulose, carboxymethycellulose, hydroxymethycellulose, elastomers, polyethylenes, polypropylenes, polybutyrate, polyacrylate, polyacrylamide, polybuterate, polyurethane foam, silicone elastomer, nylon, vinyl or cross linked dextran. If cross-linked dextran is used, it is preferred that the molecular weight of the dextran polymer is between 50,000 and 500,000. The polymeric structure of the scaffolding of the invention may either be of a cross-linked or non-cross linked polymer. A preferred embodiment is composed of a cross-linked polymer of the polyacrylate family of polymers. A most preferred embodiment is cross linked polyacrylamide, as it possesses carrying capacity for moisture, active agents and humectant and is non-degradable so that it will persist through the application and treatment phase. An example of such a polyacrylamide matrix is taught by U.S. Patent No. 5,196,190 to Nangia, et al.

Also disclosed is a novel anti-fungal agent. The present composition may be used for the delivery of one or more of the known or contemplated anti-fungal agents, provided that the one or more agents are capable of distribution via diffusion in an aqueous gradient. In addition to known therapeutic anti-fungal compounds, it is known that many other substances are capable of inhibiting or killing dermatophytes. In certain in vitro culture methods, it has been found that increasing the acidity is useful in controlling the growth of dermatophyte fungi. Low pH (i.e., high hydrogen ion concentration) prevents the growth of dermatophytes which are the principle group of fungi responsible for onychomycosis. Also described are compositions comprising one or more acidic agents that are capable of increasing the hydrogen ion concentration of the medium. A preferred source of acidity in the composition is a weak organic acid.

It is contemplated that weak organic acids can achieve relatively low pH values in aqueous gradients yet are well tolerated when in contact with tissues. There are numerous suitable organic acids, including citric acid, sorbic acid, ascorbic acid, salicilic acid, tannic acid, succinic acid, lactic acid, pyruvic acid, alpha ketoglutaric acid, glutamic acid, acetic acid and butaric acid. Salicylic acid has both keratinolytic properties as well as being a proton donor. Other anti-mycotic active agents include, iodine, DMSO, azole derivatives, undecylenic acid, tea tree oil, and urea.

Citric acid is used as the active agent in the present invention. Citric acid has 3 carboxyl residues which have distinct pK values for dissociation of protons into the aqueous environment. Therefore the buffered pH can be maintained at a relatively low level through out the application period. Furthermore, citric acid is generally regarded as safe.

A preferred embodiment comprises sufficient citric acid to achieve and maintain a concentration between 0.1-10 % w/w citric acid in the non-viable portions of the nail apparatus after equilibration of the diffusion gradient established by the moisture delivered by the matrices of the present invention. Ideally the matrix would contain between approximately 1 and 20% citric acid to accomplish this. More preferably the matrices would possess a concentration between 5 and 15 percent with the most preferred embodiment having between 7 and 13 percent citric acid incorporated in the matrix.

An additional component of embodiments of compositions of the present invention is a moisture management system that is integral to the matrices. The moisture management system may include substances that attract and hold moisture in the matrix. Mucopolysaccharides interact with moisture, which results in hydration of their polymeric structure. Water is often referred to as being "bound" to the polysaccharide. This loose binding of water serves to enable mucopolysaccharides to provide a reservoir effect so that water can be incorporated until another drier substance can take it away. Many polysaccharides may play this role but useful ones for the purpose of the invention would be those that do not gel when exposed to excess water. Numerous polysaccharides may serve the functional purpose of the mucopolysaccharide in the present invention including acacia, distarch adipate, alginic acid, agar, arabinogalactan, carrageenan, locust bean gum, methylcellulose, eucheuma seaweed, xanthan gum. One such mucopolysaccharide is derived from the Guar tree and is referred to as guar gum. A range of guar gum between approximately 0.01 kg to 100 kg, preferably between approximately 0.1 kg to 10 kg, and most preferably between approximately 0.5 kg to 2kg is generally sufficient. Other non-gellable polysaccharides and galactomannan macromolecules include lucerne, fenugreek, honey locust bean gum, white clover bean gum and carob locust bean gum. A preferred embodiment of compositions of the invention would contain guar gum for the purpose of holding water in the substrate.

Compositions of the present invention are capable of being affixed to the site of infection or growth of the unwanted organism, and the compositions are affixed by attachment elements. The utility of the device would be approximately the same whether it were simply a matrix with the properties above or was provided with some securing capability by the attachment elements. An embodiment of the invention is a matrix which is capable of hydrating and acidifying the nail bed and which would be easy to use. Ease of use would be facilitated if the device had an attachment element of an adhesive backed tape or film that extended beyond the border of the matrix such that the adhesive could be used to wrap the toe or finger so as to secure the device to the affected area. Ideally the attachment element would have a relatively low moisture vapor transmission rate so as to aid in the retention of moisture in the matrix and occlude moisture loss due to evaporation. Notwithstanding any pattern that aids in application would be a desirable feature of the device. It is further contemplated that an attachment element also includes the use of an adhesive applied to the surface of the contact portion of the matrix so that the matrix would be securely fixed to the affected area by the adhesive, thereby preventing it from slipping away from the nail. An added advantage would be an open pattern adhesive since many adhesives are hydrophobic and only minimally allow moisture movement through the substrate. Such an open pattern adhesive would not interfere with the transfer of moisture and active agents from the matrix to the affected area being treated. The matrix could also comprise an attachment element of a tacky or adhesive material incorporated in the matrix so that the material could be molded to the affected area and would remain attached due to its inherent cohesive properties. Any attachment element that is capable of securing or maintaining the matrix at the site of application is contemplated by the present invention and many such attachment elements, for example those used for securing bandages, are known to those skilled in the art.

Examples of attachment elements include pressure sensitive adhesives including polysiloxanes (e.g., polydimethyl siloxanes, polydiphenyl siloxanes, and siloxane blends), polyisobutylenes, polyacrylates, acrylic acid-acrylate copolymers (e.g., copolymers of acrylic acid copolymers with 2-ethylhexyl acrylate or isooctyl acrylate), and tacky rubbers such as polyisobutene, polybutadiene, polystyrene-isoprene copolymers, polystyrene-butadiene copolymers, and neoprene (polychloroprene). An attachment element, such as a pressure sensitive adhesive material, with respect to highly hydrated biological tissues, such as mucosal tissue, exhibit good tack when adhered to hydrated biological substrates. To be bioadhesive, water should provide a plasticizing effect on the polymer, i.e., the polymer should be hydrophilic. For example, the range of typical adhesives includes slightly cross-linked polyacrylic and polymethacrylic acids (EP 0371421) as well as blends of hydrophilic cellulose derivatives (40-95%) with polyethylene glycol (PEG) (U.S. Pat. No. 4,713,243). Bioadhesives become tacky as the cross linked polymer swells in significant quantities of water. Such adhesives are taught in U.S. Patent No. 6,576,712.

In the present invention, it is contemplated that embodiments of compositions of the delivery platform would function on contact with the nail plate to deliver aqueous moisture that will increase the hydration level in the nail plate. The organic acid in the material would then move from the delivery platform to establish an even distribution of acidity throughout the device and the nail plate and other non-viable tissues of the nail apparatus.

A device with the above properties comprises a sheet of matrix material that could be cut to the size and shape of finger and toe nails. The material is applied directly to the nail with attachment elements provided by the tackiness of the polymer of the matrix to hold it in place and/or by attachment elements such as an adhesive polymer coating or adhesive backing securing device. Such a device is worn for several days, is preferably transparent which has cosmetic appeal, and maintains the moisture gradient due to the humectant or water retention property of the material.

Compositions of the present invention comprise citric acid and may comprise other active agents. Such active agents include organic acids, and antifungal agents such as selenium sulfide, resorcinol, ketoconazole (NIZORAL) Clotrimazole (such as LOTRlMIN), Terbinafine (LAMISIL), Ciclopirox olamine (LOPROX), Diflucan, anti-yeast compounds, antibacterial compounds, and antiviral compounds. Three alternative formulations of a topical delivery composition not according to the present invention as described in general terms below.

### Formulation 1

Active ingredient, such as citric acid, at preferred percentage

| | |
|---|---|
| Glycerin | 2.0% w/v |
| Propylene Glycol | 2.0% w/v |
| Dimethylisosorbide | 0.5% w/v |
| Ethanol | 35.00% w/v |
| Preservatives | as needed |
| Water | to final volume |

### Formulation 2

Active ingredient, such as citric acid, at preferred percentage

| | | |
|---|---|---|
| Glycerin | 2.40% w/v | |
| Propylene Glycol | 0.60% w/v | |
| Dioctyl Maleate | | 1.00% w/v |
| Ethanol | | 28.50% w/v |
| Preservatives | | as needed |
| Water | | to fmal volume |

### Formulation 3

Active ingredient, such as citric acid, at preferred percentage

| | | |
|---|---|---|
| Caprylic/Capric | | 0.25% w/v |
| Triglyceride | | |
| Propylene Glycol | 2.00% w/v | |
| Dimethylisosorbide | | 1.00% w/v |
| Ethanol | | 40.00% w/v |
| Pantoethanol | | 0.25% w/v |
| Preservatives | | as needed |
| UV A, B and other | as needed | |
| radiation screens | | |
| Free radical inhibitors | as needed | |
| or quenchers | | |
| Water | | to final volume |

Compositions of the present invention may be used for treatment of dermal structures by applying a composition that is effective in the prevention of growth or infection of unwanted organisms until the growth or infection by the unwanted organisms is lessened or stopped. For example, a treatment of a dermal structure comprising, a) applying a composition comprising at least one active agent to a site of a dermal condition, b) maintaining the composition at the site for a sufficient amount of time so that an effective amount of the active agent is delivered.

Such uses may further comprise treatment of the entire host organism by the concomitant use of oral medications that aid in the prevention or cessation of the growth or infection by the unwanted organisms. Such oral treatments include antifungal pills. Antifungal pills may be taken just once (in a single dose) or may be taken once a day for 5 to 10 days, or continuously. A short period of time of dosage will mitigate against the side effects or risks usually associated with long-term use. Antifungal pills are available include Ketoconazole (NIZORAL), Fluconazole (DIFLUCAN), Griseofulvin and Itraconazole (SPORANOX).

Dermal conditions that may be treated by the compositions of the present invention include Acne, Grover's Disease, Pityriasis Lichenoides, Acanthosis Nigricans, Hair Loss (Alopecia Areata), Pityriasis, Rosea, Acrochordons, Hair Loss (Androgenic Alopecia), Pityriasis, Rubra, Pilaris, Actinic Keratosis, Hair Loss (Telogen Effluvium), Plantar Warts, Age Spots, Halo Nevus, Poison Ivy, Allergic Contact Dermatitis, Hand Dermatitis, Poison Oak, Anal Warts, Heat Rash, Pompholyx, Angioma, Herpes Simplex, Pre-cancers of the Skin, Aphthous Ulcers, Herpes Zoster (Shingles), Pruritus Ani (Itchy Butt), Athlete's Foot, Hidradenitis Suppurativa, Pseudofolliculitis Barbae, Atopic Dermatitis, Hives, Psoriasis, Atypical Moles, Hyperhidrosis, Razor Bumps, Barnacles of Aging, Ichthyosis, Rhus Allergy, Basal Cell Carcinoma, Impetigo, Rhyniophyma, Bateman's Purpura, Ingrown Hairs, Ring Worm (Body), Berloque Dermatitis, Irritant vs. Allergic Dermatiti, Ring Worm (Scalp) Boils, Jock Itch, Bruising Back of Arms, Keloids, Scabies, Bullous Pemphigoid, Keratoacanthoma, Scar, Abnormal Candida, Keratosis Pilaris, Schamberg's Disease, Carbuncles and Furuncles, Lentigines (Sun Spots), Scleroderma, Localized Cherry Angioma, Lichen Planus, Sebaceous Hyperplasia, Chiggers, Chondrodermatitis Helicis, Lichen Simplex Chronicus, Seborrheic Keratosis, Clark's Nevus, Lichen Sclerosus, Senile Angioma, Cold Sores, Lichen Striatus, Condylomata, Lupus of the Skin, Skin Aging Cysts, Lyme's Disease, Skin Tags, Dandruff, Lymphomatoid Papulosis, Solar Keratosis, Mask of Pregnancy, Squamous Cell Carcinoma, Darier's Disease, Melanoma, Stasis Dermatitis, Dermatofibroma, Melasma, Sun Bum, Diaper Dermatitis, Miliaria, Sun Damage, Discoid Lupus Erythematosus, Moles, Sun Spots, Dry Skin, Molluscum Contagiosum, Dyshidrotic Dermatitis, Mycosis Fungoides, Telogen Effluvium, Eczema, Atopic Myxoid Cysts, Tinea Capitis, Dyshidrotic, Nail Splitting, Brittle, Tinea Corporis, Nail Fungus, Tinea Cruris, Necrobiosis Lipoidica Diabeticorum, Tinea Pedis, Nickel Allergy, Tinea Versicolor, Erythema Multiforme, Nummular Dermatitis, Urticaria, Erythema Nodosum, Onychomycosis, Urticaria Pigmentosa, Folliculitis, Onychoschizia, Vitiligo, Folliculitis Keloidalis Nuchae, Perioral Dermatitis, Warts, Fordyce's Condition, Pfiesteria, Xanthomas, Granuloma Annulare, Pimples, Xerosis (Dry Skin), Pityriasis Alba, and yeast infection.

The foregoing description includes the best presently contemplated mode of carrying out the invention. This description is made for the purpose of illustrating the general principles of the inventions. This invention is further illustrated by the following examples .

All terms used herein are considered to be interpreted in their normally acceptable usage by those skilled in the art.

### EXAMPLE 1

### Formation of a Matrix Including Acrylamide

A mixing tank was charged with 161.4 kg of water and 9.1894 kg of acrylamide, 0.10347 kg of NNNN'-methylenebisacrylamide, and 9.3046 kg of glycerol were added and mixed. Then 1.0213 kg of guar gum non-gellable polysaccharide was dispersed in a mixture containing 0.9770 kg of isopropyl alcohol and 2 kg of water. The solution of guar gum was then added and dispersed into the acrylamide mixture. After suitable mixing, 0.1042 kg of TEMED was added and polymerization was catalyzed with 0.0999 kg ammonium persulphate.

While the batch was still liquid, it was poured into molds to form sheets. After gelling had occurred, sheets were transferred to a dessicator and dehydrated to form a stable intermediate stock sheet. Prior to cutting to size, the stock material was re-hydrated in a humid atmosphere. After cutting, the material was coated with petrolatum. The resulting composition was then sealed into appropriate packaging and irradiated to sterilize it.

An embodiment of the invention is a hydrogel matrix composed of a cross-linked polyacrylamide scaffolding containing ingredients that assist in the development and maintenance of an aqueous diffusion gradient in the nail plate. The matrix used in the prototypes utilized the formulation based on the Nangia patent (US 5196390), such as that described above. The matrix was made by dissolving the acrylamide, bis acrylamide, guar gum, glycerol and citric acid in the aqueous charge and then initiated and catalyzed polymerization with TEMED and sodium persulfate. The resulting gel was cross linked hydrogel polymer with aqueous moisture, glycerol and citric acid. The concentration of citric acid in the formulation was approximately 6 to 16% w/w after the adjustment of the final water content to approximately 50% by weight of the matrix using a dehumidifier.

### EXAMPLE 2

An alternative method for making a polyacrylamide matrix containing citric acid as the active agent was performed. The steps of Example 1 were followed, except the citric acid was not added. Once the matrix was polymerized, it was formed into the desired shape, typically a sheet approximately 25 x 25 x 0.5 cm in size. A sheet of hydrophilic matrix was created by dehydration at 45°C resulting in approximately 3% w/w moisture. The sheet was then reconstituted with the addition of concentrated solutions of citric acid to form sheets so that the moisture content was approximately 50% by weight. The concentration of citric acid in the prototypes was 6%, 8%, 10%, 12 % and 16% by weight. The polymerized matrices with these concentrations of acid retained the normal properties of plain matrix material and were suitable for application. Sheets made by this procedure were sealed into medical grade polyethylene pouches until used.

### EXAMPLE 3

The application of the sheet to infected nails was carried out by cutting the matrix to the approximate size of the nail. This was then placed onto the nail and secured using a medical grade polyurethane adhesive thin film dressing (OPSITE, Smith & Nephew). The cover dressing was applied so that the matrix parts were completely boardered on all sizes. This prevented slippage of the matrix from the nail but also occluded air contact with the matrix which might lead to dehydration. Matrix applied in this fashion could be worn for up to one week but typically were changed approximately every 2-3 days.

All prototypes, regardless of the concentration of citric acid were effective at hydrating the nail and particular the portion directly over active fungal invasion (as apparent by the white discoloration of the nail). Several individuals that volunteered to wear samples of the various strengths complained of burning sensation when the 16% sample was worn. This also occurred occasionally when the 12% sample was worn. However discontinuation resulted in relief of the sensation. Moreover individuals that experienced irritation with a higher concentration could all wear the 8% concentration without irritation.

After 1-2 applications, the portion of nail affected by fungi completely detached from the nail bed and was free floating. This detached portion could then be removed by scissors or clipper so that subsequent applications of the invention were in direct contact with the nail bed where the fungi typically reside.

Typically nail bed areas took on nearly normal appearance after about 14 days of application. Interestingly, the matrix has no apparent affect on healthy nail so that there may be no disadvantage in prolonged application where there is a high risk of re-infection from the surrounding area which often happens with people that have co-incident athletes' foot infections.

Citric acid containing matrix was used in one case of an individual with affected 7 toes (4 of the left foot and 3 on the right foot). Matrix was regularly applied every other day for 21 days to the nails of the left foot. All of the nails were hydrated within the first application. The detached nail plate substrate over the areas infected were debrided and application continued. New nail growth was observed by the 10^{th} day and continued until normal nail length was achieved. No changes were observed in the affected untreated nails of the right foot.

### EXAMPLE 4

### Skin Sensitivity

Matrices were constructed with varying amounts of citric acid, glycerol and water, as described in Examples 1 or 2. These matrices were screened for irritation and sensitization by application to the skin surface of volunteers. The prototypes and exposure results are laid out in the Table 2. CA=citric acid.

**Table 2. Skin irritation and sensitization reactions on human volunteers.**

| Prototype | Composition | Irritation Reaction (+/-) |
|---|---|---|
| A.1 | Matrix containing 8% CA, 25% glycerol and 25% water. | 0/5 |
| A.2 | Matrix containing 8%CA, 15 % glycerol and 50% water | 0/5 |
| A.3 | Matrix containing 12% CA, 25% glycerol and 25% water. | 1/7 |
| A.4 | Matrix containing 12% CA, 15% glycerol and 50% water. | 0/5 |
| A.5 | Matrix containing 16% CA, 25% glycerol and 25% water. | 3/5 |
| A.6 | Matrix containing 16% CA, 15% glycerol, and 50% water. | 2/4 |

The occurrence of undesirable reactions at the application sites where matrices containing 16% citric acid caused the discontinuation of investigation of materials containing this amount of acid.

### EXAMPLE 5

### Inhibition of Fungal Growth

Antifungal activity was determined by growth inhibition using a modified zone inhibition type assay. Strains of fungi were propagated on Sabouraud's Dextrose Agar until confluent. *Aspergillus niger* spores were harvested by rinsing culture with saline containing Tween 20. Hyphal growth from dermatophytes was also harvested using saline containing Tween 20. Hyphal growth was transferred to 15 ml sterile plastic tubes with 10 ml Tween and 3-4 glass beads. The tubes were shaken for about 2-3 minutes by hand to break up hyphae. Aliquots of 1.5 ml of hyphae or spore suspensions were dispensed to the surface of Sab-Hi agar medium (Sabouraud's containing Trypticase Soy Broth). The suspensions were spread evenly over the surface and then excess fluid was decanted and discarded. Samples of citric acid-containing, and control matrices as described in Examples 1 and 2 were prepared by punch cutting using a 5 mm bore punch. The samples were transferred to the plates and pressed to ensure contact with the plates. The plates were then incubated at 30°C in a humidified atmosphere for 3-5 days to allow fungal growth. The plates were observed for overt appearance of zones of inhibition and then microscopically under the specimens for mycelial invasion into the contact area.

The specimens that were tested in the system were the control (HexiGel Hydrogel Wound Dressing (plain matrix)), matrix with 8% citric acid (50% hydrated) and matrix with 12% citric acid (again 50% hydrated). The test organisms were Trichophyton rubrum, Epidermophyton, Microsporum and Aspergillus niger. The results are provided in Table 3.

**Table 3. Prototype action on the growth of fungi on solid agar medium.**

| [Diameter of Zone / Growth of fungi in contact area] | | | | | | |
|---|---|---|---|---|---|---|
| Test Organism | FlexiGel Control | | Matrix 8% CA | | Matrix 12% CA | |
| Aspergillus niger | 0 mm | Heavy | 0 mm | Heavy | 0 mm | Heavy |
| Trichophyton | 0 mm | Heavy | 5 mm | None | 9 mm | None |
| Epidermophyton | 0 mm | Heavy | 2 mm | None | 4 mm | None |
| Microsporum | 0 mm | Heavy | 9 mm | None | 12 mm | None |

These results showed that Aspergillus was not inhibited by either of the test samples. Hyphae from this fungi grew up to, over, as well as under the citric acid containing matrices. This is not surprising since Aspergillus is commonly isolated from citrus fruits and in fact is used commercially to ferment cane sugar, molasses and dextrose into citric acid. The specimens containing citric acid inhibited all of the strains of dermatophytes. The zones of inhibition were greatest with Trichophyton and Microsporum Continued incubation of all of the plates eventually resulted in hyphal invasion of the inhibition zones of the dermatophytes. This was likely to be due to the equilibration of citric acid into the medium resulting in a decreased concentration in the immediate vicinity of the test specimens. At the time of scanning (day 4 post inoculation) no hyphae were detected under the CA containing samples placed on the dermatophytes. By contrast, heavy hyphal growth was present under the Control sample as well as under all specimens tested on Aspergillus.

### EXAMPLE 6

### Transfer of Citric Acid Through Nail Plate

Effective inhibition of the dermatophytes responsible for nail infections require the penetration of the citric acid to the junction between the nail bed and the nail plate. A model of delivery was made by preparing "artificial" nail plate from bovine hoof.

Preparation of bovine hoof: Beef hoof obtained from an abitor was cleaned with soap and water and then air dried. The hoof was then mounted in a clamp device to secure it so that uniform shavings, which were small slabs of hoof, could be taken using a hand plane. The shavings were taken parallel to the surface of the face of the hoof. The shavings were collected and saved at 4°C until required.

Transfer of acidity. The transfer of acidity from the prototypes was evaluated by placing matrix with citric acid on top of a hoof shaving which was on top of a control matrix sample. In theory, the accumulation of acid in the control matrix would only result if it passed through the hoof. To confirm this, similar set-ups were made except that a layer of polyethylene sheet was also placed between the control and test matrix to confirm that passage "through" the hoof occurred. Acid accumulation was shown by the measure of pH change, phenol red conversion, and citric acid detection (pyridine-acid anhydride test) after overnight incubation at room temperature. The results are in Table 4.

**Table 4. Acid transfer through keratinized beef hoof simulation of nail plate.**

| Sample Tested | pH | Phenol Red | Pyridine |
|---|---|---|---|
| FlexiGel Control alone | 6.5 | 2mm | Clear |
| 12% Citric Acid alone | 3.0 | 21mm | Dark Red |
| FlexiGel below 12% CA | 4.0 | 10 mm | Light yellow |
| FlexiGel below 12% CA separated by poly film | 6.0 | 2mm | Clear |

The results of this study showed that protons migrate readily through the hoof to the basal layer. The migration was not around the hoof shaving because it is blocked by a plastic impermeable barrier. The amount of citrate that was delivered to the underlying substrate is less than the amount in the delivery substrate.

### EXAMPLE 7

### Penetration of Acidity Through Human Nail Plate

A sample of nail clipping was taken from a volunteer as a control. The volunteer then applied the 12% CA matrix for overnight wear to a toe. The next day an additional clipping was taken from the treated toe nail. Both specimens were then oriented so that ventral aspect of each clipping was situated against the surface of a phenol red agar plate. The plate was then incubated for 2 hours at room temperature to determine if acidity penetrated through the nail plate. The untreated nail clipping showed no change in color of the surrounding phenol red whereas the treated nail clipping had an area approximately one inch in diameter surrounding it with all red cleared, indicating acidification. This simple test showed that acidity penetrated rapidly through the hydration gradient created in the nail during the treatment.

The above studies showed that the agent and delivery means are effective in the treatment of onychomycosis for the vast majority of cases since dermatophytes cause as much as 96% of the cases.

## Claims

1. A composition for use in treating nail fungal infections, the composition comprising
a) a matrix sheet comprising a natural or synthetic hydrophilic polymer;
b) one or more active agents, wherein at least one active agent is citric acid;
c) at least one humectant; and
d) a water content of 0.1-50%.

2. A composition for use according to claim 1, wherein the one or more active agent is citric acid and sorbic acid, ascorbic acid, salicylic acid, tannic acid, succinic acid, lactic acid, pyruvic acid, alpha ketoglutaric acid, glutamic acid, acetic acid, butyric acid, salicylic acid iodine, DMSO, undecylenic acid, tea tree oil, urea, selenium sulfide, resorcinol, ketoconazole, Clotrimazole, Terbinafme, Ciclopiroxolamine, Diflucan, anti-yeast compounds, antibacterial compounds, or antiviral compounds.

3. A composition for use according to claim 1 or claim 2, wherein the humectant is an organic alcohol, glycerol, butanol, propanol, isopropyl alcohol, ethanol, methanol, propylene glycol, polyethylene glycol, ethylene alcohol, butyl alcohol, sodium chloride, lithium chloride, copper chloride, magnesium chloride, magnesium sulfate, manganese sulfate, aluminum sulfate, zinc sulfate, or zinc chloride.

4. A composition for use according to any of claims 1 to 3, wherein the natural or synthetic hydrophilic polymer is rubber, collagen, animal hide, hyaluronic acid, dextran, alginates, cellulose, carboxymethycellulose, hydroxymethycellulose, elastomers, polyethylenes, polypropylenes, polybutyrate, polyacrylate, polyacrylamide, polybuterate, polyurethane foam, silicone elastomer, nylon, vinyl or cross-linked dextran.

5. A composition for use according to any preceding claim, further comprising a moisture management system.

6. A compositions for use according to any preceding claim, further comprising an attachment element.

7. A composition for use according to any preceding claim, wherein the citric acid is in a concentration between 0.1 % and 16%.

8. A composition for use according to claim 1, the composition comprising a cross-linked polyacrylamide matrix sheet, glycerol, a non-gellable polysaccharide, citric acid, and water, wherein the citric acid is in a concentration range of from 8% to 16% w/w, and the water content is 50%.

9. Use of citric acid in the preparation of a medicament comprising a matrix sheet comprising a natural or synthetic hydrophilic polymer, at least one humectant and water content of 50%, for the treatment of nail fungal infections.

10. The use according to claim 9, wherein the polymer is cross-linked.

## Patentansprüche

1. Zusammensetzung für die Verwendung bei der Behandlung von Nagelpilzerkrankungen, wobei die Zusammensetzung enthält:
a) eine Matrixlage, umfassend ein natürliches oder synthetisches hydrophiles Polymer;
b) eines oder mehrere aktive Agenzien, wobei zumindest ein aktives Agens Zitronensäure ist;
c) zumindest ein Feuchthaltemittel und
d) einen Wassergehalt von 0,1-50%.

2. Zusammensetzung für die Verwendung gemäß Anspruch 1, wobei das eine oder die mehreren aktiven Agenzien Zitronensäure und Sorbinsäure, Ascorbinsäure, Salicylsäure, Tanninsäure, Succinylsäure, Milchsäure, Brenztraubensäure, Alpha-Ketoglutarsäure, Glutaminsäure, Ethansäure, Butansäure, Salicylsäure-Jod, DMSO, Undecylensäure, Teebaumöl, Harnstoff, Seleniumsulfid, Resorzinol, Ketoconazol, Clotrimazol, Terbinafin, Ciclopiroxalmin, Diflucan, Antiyeast-Verbindungen, antibakterielle Verbindungen oder antivirale Verbindungen.

3. Zusammensetzung für die Verwendung gemäß Anspruch 1 oder Anspruch 2, wobei das Feuchthaltemittel ein organischer Alkohol, Glycerin, Butanol, Propanol, Isopropylalkohol, Ethanol, Methanol, Propylenglycol, Polyethylenglycol, Ethylenalkohol, Butylalkohol, Natriumchlorid, Lithiumchlorid, Kupferchlorid, Magnesiumchlorid, Magnesiumsulfat, Mangansulfat, Aluminiumsulfat, Zinksulfat oder Zinkchlorid ist.

4. Zusammensetzung für die Verwendung gemäß einem der Ansprüche 1 bis 3, wobei das natürliche oder synthetische hydrophile Polymer Gummi, Collagen, Tierhaut, Hyaluronsäure, Dextran, Alginate, Cellulose, Carboxymethylcellulose, Hydroxymethylcellulose, Elastormeren, Polyethylen, Polypropylen, Polybutyrat, Polyacrylat, Polyacrylamid, Polybuterat, Polyurethanschaum, Silikonelastomer, Nylon, Vinyl oder quervernetztes Dextran ist.

5. Zusammensetzung für die Verwendung gemäß einem der vorhergehenden Ansprüche, ferner umfassend ein Feuchtigkeits-Managementsystem.

6. Zusammensetzung für die Verwendung gemäß einem der vorhergehenden Ansprüche, ferner umfassend ein Anhaftungselement.

7. Zusammensetzung für die Verwendung gemäß einem der vorhergehenden Ansprüche, wobei die Zitronensäure in einer Konzentration zwischen 0,1 % und 16% vorliegt.

8. Zusammensetzung für die Verwendung gemäß Anspruch 1, wobei die Zusammensetzung eine quervernetzte Polyacrylamid-Matrixlage, Glycercol, ein nichtgelierendes Polysaccharid, Zitronensäure und Wasser umfasst, wobei die Zitronensäure in einem Konzentrationsbereich von 8% bis 16% w/w enthalten ist und der Wassergehalt 50% beträgt.

9. Verwendung von Zitronensäure in der Herstellung eines Medikaments, umfassend eine Matrixlage, die ein natürliches oder synthetisches hydrophiles Polymer, zumindest ein Feuchthaltemittel enthält und einen Wassergehalt von 50% aufweist, für die Behandlung von Nagelpilzerkrankungen.

10. Verwendung gemäß Anspruch 9, wobei das Polymer quervernetzt ist.

## Revendications

1. Composition destinée à être utilisée pour le traitement des affections fongiques de l'ongle comprenant :
a) une feuille matrice renfermant un polymère hydrophile naturel ou synthétique,
b) au moins un agent actif, cet agent actif étant l'acide citrique,
c) au moins un agent humidifiant, et
d) une teneur en eau de 0,1 - 50 %.

2. Composition destinée à être utilisée conformément à la revendication 1, dans laquelle l'agent actif est l'acide citrique et l'acide sorbique, l'acide ascorbique, l'acide salicylique, l'acide tannique, l'acide succinique, l'acide lactique, l'acide pyruvique, l'acide alpha cétoglutarique, l'acide glutamique, l'acide acétique, l'acide butyrique, l'acide salicylique, l'iode, le DMSO, l'acide undécylénique, l'huile de théier, l'urée, le sulfure de sélénium, le résorcinol, le cétoconazole, le Clotrimazole, la Terbinafine, la Ciclopiroxolamine, le Diflucan, les composés anti-levures, les composés anti bactériens ou les composés anti viraux.

3. Composition destinée à être utilisée conformément à la revendication 1 ou à la revendication 2, dans laquelle l'agent humidifiant est un alcool organique, le glycérol, le butanol, le propanol, l'alcool iso-propylique, l'éthanol, le méthanol, le propylène glycol, le polyéthylène glycol, l'éthylène alcool, l'alcool butylique, le chlorure de sodium, le chlorure de lithium, le chlorure de cuivre, le chlorure de magnésium, le sulfate de magnésium, le sulfate de manganèse, le sulfate d'aluminium, le sulfate de zinc ou le chlorure de zinc.

4. Composition destinée à être utilisée conformément à l'une des revendications 1 à 3, dans laquelle le polymère hydrophile naturel ou de synthèse est le caoutchouc, le collagène, la peau animale, l'acide hyaluronique, le dextrane, les alginates, la cellulose, la carboxy-méthyl cellulose, l'hydroxy méthyl cellulose, les élastomères, les polyéthylènes, les polypropylènes, le poly butyrate, le polyacrylate, le poly acrylamide, le poly butyrate, la mousse de polyuréthane, les élastomères de silicone, le nylon, le vinyle ou le dextrane réticulé.

5. Composition destinée à être utilisée conformément à l'une des revendications précédentes, comprenant en outre un système de contrôle de l'humidité.

6. Composition destinée à être utilisée conformément à l'une des revendications précédentes, comprenant en outre un élément de fixation.

7. Composition destinée à être utilisée conformément à l'une des revendications précédentes, dans laquelle l'acide citrique est présent à une concentration comprise entre 0,1 % et 16 %.

8. Composition destinée à être utilisée conformément à la revendication 1, comprenant une feuille matrice en poly acrylamide réticulé, du glycérol, un polysaccharide non gélifiable, de l'acide citrique et de l'eau, l'acide citrique étant présent dans une plage de concentration pondérale comprise entre 8 % et 16 % et la teneur en eau étant de 50 %.

9. Utilisation de l'acide citrique pour la préparation d'un médicament comprenant une feuille matrice renfermant un polymère hydrophile naturel ou synthétique, au moins un agent humidifiant et une teneur en eau de 50 % pour le traitement des affections fongiques de l'ongle.

10. Utilisation conforme à la revendication 9, dans laquelle le polymère est réticulé.
